# EUROPEAN PATENT APPLICATION

(11) **EP 3 333 574 A1**
(43) Date of publication of application: **13.06.2018**
(21) Application number: 16832838.3
(22) Date of filing: 26.07.2016
(51) Int. Cl.: G01N 33/52, G01N 33/48, G01N 33/84

(54) **ENVIRONMENT-RESPONSIVE DYE-ACCUMULATED NANO PARTICLES, AND METHOD FOR ANALYZING INTRACELLULAR ENVIRONMENT**

(30) Priority: 05.08.2015 JP 2015154942
(71) Applicant: Konica Minolta, Inc., Tokyo 100-7015 (JP)
(72) Inventor: AIMIYA, Takuji, Tokyo 100-7015 (JP); FURUSAWA, Naoko, Tokyo 100-7015 (JP)
(74) Representative: Curran, Clair
(86) International application number: PCT/JP2016/071812
(87) International publication number: WO 2017/022559

(57) **Abstract**

An object of the invention is to provide an environmentally-responsive dye-accumulated nanoparticle for grasping an intracellular environment such as pH with respect to each cell. The invention provides an environmentally-responsive dye-accumulated nanoparticle, in which an environmentally-responsive dye is accumulated on the surface of, or inside a thermosetting resin particle, and which has an average particle diameter of 30 to 800 nm, wherein the accumulated amount of the environmentally-responsive dye is 200 to 10,000,000 molecules/particle. The environmentally-responsive dye has dependence on pH, or a Ca ion in terms of fluorescence intensity, and the thermosetting resin is a melamine resin.

## Description

### Technological Field

The present invention relates to an environmentally-responsive dye-accumulated nanoparticle, and a method for analyzing intracellular environment using the same.

### Background

A pathological diagnosis is conducted as one of medical diagnoses. As a pathological diagnosis, immune surveillance is conducted, by which molecular target staining called as immunostaining for ascertaining expression of molecular information of a sample is performed to diagnose dysfunction such as abnormal expression of a gene or a protein. A cytodiagnosis or a cytological diagnosis has been conducted as one of pathological diagnoses, by which a cell itself is observed to identify an abnormal cell, etc. for seeking a pathological diagnosis or a clinical diagnosis concerning existence or nonexistence of a lesion or a lesion site.

Inside a cell, there are cell organelles, such as ribosome, endoplasmic reticulum, Golgi body, mitochondrion, and lysosome, besides a nucleus in the cytoplasmic matrix. It is believed that there exists a characteristic environment of a limited part in a cell corresponding to a specific organelle. It is therefore expected greatly that such an intracellular environment, once it can be grasped, would contribute to detection of lesion of a cell itself, or application to a cytological diagnosis and accuracy improvement thereof.

However, absolutely no means able to evaluate such an intracellular environment has been proposed.

Patent Document 1 (JP 4444363 B) discloses a layered-structure silica nanoparticle containing a fluorescent dye molecule, or a light-absorbing dye, and a labeling reagent using the silica nanoparticle. Patent Document 1 discloses further that elimination of a functional molecule from a silica nanoparticle may be suppressed by forming a layered structure so as to improve the intake efficiency of a functional molecule into a silica particle.

Patent Document 2 (JP 2010-508295 W) discloses a novel fluorescent dye responsive to pH. The Patent Document 2 discloses a fluorescent dye responsive to pH bonded covalently to a polymer fine particle, which is a carrier molecule.

In this regard, Patent Document 1 is related to high sensitivity quantitative analysis of an infinitesimal quantity of target substance, and Patent Document 2 has a purpose of detection of phagocytosis.

### Related Art Document

### Patent Documents

Patent Document 1: JP 4444363 B
Patent Document 2: JP 2010-508295 W

### Summary

### Technical Problem

Using Patent Document 1 for reference a silica nanoparticle containing a pH-responsive dye, and using Patent Document 2 for reference a pH-responsive dye-binding polystyrene particle were synthesized, and pH measurements inside cultured cells were tried to find that a region where fluorescence was observed in any of particles had a size in the order of micrometer, and larger than the particle size. Further, the fluorescence intensity was so low that observation of a nano-sized region (size equivalent to a single particle) was not possible, let alone analysis of the environment inside a cell.

### Solution to Problem

In such a situation, the inventors have developed a novel environmentally-responsive dye-accumulated nanoparticle, which enables observation of a nano-sized region (size equivalent to a single particle).

The constitution of the present invention is as follows.
[1] An environmentally-responsive dye-accumulated nanoparticle, in which an environmentally-responsive dye is accumulated on the surface of, or inside a thermosetting resin particle, and which has an average particle diameter of 30 to 800 nm, wherein the accumulated amount of the environmentally-responsive dye is 200 to 10,000,000 molecules/particle.
[2] The environmentally-responsive dye-accumulated nanoparticle according to [1] above, wherein the environmentally-responsive dye has dependence on pH, or a Ca ion in terms of fluorescence intensity.
[3] The environmentally-responsive dye-accumulated nanoparticle according to [1] or [2] above, wherein the thermosetting resin is a melamine resin.
[4] The environmentally-responsive dye-accumulated nanoparticle according to any one of [1] to [3] above, wherein the surface is further modified with poly(ethylene glycol).
[5] A reagent for an intracellular environment analysis comprising the environmentally-responsive dye-accumulated nanoparticle according to any one of [1] to [4] above.
[6] A method for analyzing intracellular environment, wherein the environmentally-responsive dye-accumulated nanoparticle according to any one of [1] to [4] above is introduced into a cell, and the inside of the cell is evaluated.

### Advantageous Effects of Invention

According to the present invention an intracellular environment such as pH may be grasped with respect to each cell. Therefore, it may be highly expected that the above should contribute to detection of lesion in a cell itself, as well as application to a cytological diagnosis and accuracy improvement thereof.

### Description of Embodiments

An embodiment of the present invention will be described, provided that the present invention be not interpreted in a limited way by reason of the embodiment.

### «Environmentally-responsive dye-accumulated nanoparticle»

An environmentally-responsive dye-accumulated nanoparticle according to the present invention is formed by accumulating an environmentally-responsive dye on the surface of, or inside a thermosetting resin particle to be used as a base material. In this regard, a "dye-accumulated nanoparticle" means a resin particle to be used as a base material, on or in which dye molecules are accumulated by means of a chemical bond, an electrical attractive force, physical inclusion, adsorption, or the like.

### Thermosetting resin particle

Examples of a thermosetting resin constituting a nanoparticle may include polystyrene, polyamide, poly(lactic acid), polyacrylonitrile, poly(glycidyl methacrylate), polymelamine, polyurea, polybenzoguanamine, polyfuran, polyxylene, and a phenol resin. Although there is no particular restriction on a thermosetting resin constituting a particle insofar as it may be able to retain stably an environmentally-responsive dye, a melamine resin is preferable. Since a melamine resin is able to accumulate a dye by means of a covalent bond or an electrostatic bond, it is superior in accumulation capacity. Especially in the case of an electrostatic bond, dye particles repel each other, so that aggregation of dye particles may be inhibited and the quantum yield may be improved.

The average particle diameter of an environmentally-responsive dye-accumulated nanoparticle, namely the average particle diameter of a thermosetting resin particle to be used as a base material is in a range from 30 to 800 nm, and preferably from 50 to 500 nm. Since the size of a somatic cell is ordinarily in a range of several tens of micrometers to several hundreds of micrometers, when the average particle diameter is in the above range, intake of a nanoparticle into a cell in such an amount as enabling identification of the environment becomes possible. Further, there is no particular restriction on the coefficient of variation indicating fluctuation of a particle diameter, and it is ordinarily 20% or less, and preferably 5 to 15%. When the average particle diameter of a nanoparticle is below the range, the accumulated amount of a dye becomes low, and therefor light emission may become insufficient, and when it is above the range, the particle is too large compared to a cell or a cell organelle, minute environment changes may not be recognized.

The particle diameter of an environmentally-responsive dye-accumulated nanoparticle may be measured as the diameter of a circle having an area equal to a measured area of a cross section (area equivalent circle diameter) of an environmentally-responsive dye-accumulated nanoparticle, obtained by taking an electron micrograph using a scanning electron microscope (SEM). The average of the particle sizes of a group of environmentally-responsive dye-accumulated nanoparticles (average particle diameter) and the coefficient of variation of the same are obtained after measurements as above of the particle sizes (particle diameter) for a sufficient number (for example, 1000) of environmentally-responsive dye-accumulated nanoparticles, and with respect to the average particle diameter by calculating an arithmetic mean of the above, and with respect to the coefficient of variation by calculation according to the formula: (100×standard deviation of particle diameters) / (average particle diameter).

### Environmentally-responsive dye

An environmentally-responsive dye means a dye, which emits fluorescence or changes fluorescence intensity responding to a change in an environment, such as pH, metal ion, gas concentration, temperature, and pressure. A nanoparticle containing an environmentally-responsive dye emits fluorescence. Among others, a dye having responsiveness to pH, or a Ca ion is desirable for assessing the environment in a cell according to the present invention.

As such an environmentally-responsive dye, those described in JP 2003-501540 W, or JP 2010-508295 W may be used.

A dye described in JP 2010-508295 W is, for example, a compound expressed by the following Formula A, in which R¹ and R² are none of hydroxy, thiol, and a deprotonated form thereof. 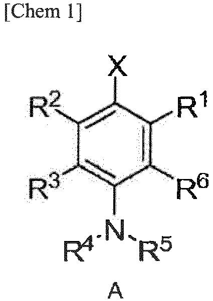
(wherein, R¹ to R⁶ are hydrogen, or a substituent other than an electron-withdrawing group, and X is a fluorophore; or
R¹ and R³ to R⁶ are hydrogen, or a substituent other than an electron-withdrawing group, and X and R² form together a moiety containing a 5-membered or 6-membered heterocyclic ring condensed to a benzene ring of Formula A between positions X and R²).

In this regard, a "fluorophore" means a composition, which is innately fluorescent, or shows a change in fluorescence on an occasion of protonation, bonding to a biological compound, or a metal ion, or enzymatic metabolism. Examples of a preferable fluorophore may include xanthene, indole, borapolyazaindacene, furan, and benzofuran.

Specific examples of a dye compound expressed by Formula A are as disclosed in JP 2010-508295 W. The dyes have responsiveness to a Ca ion.

Dyes described in JP 2003-501540 W are, for example, expressed by the following Formula (I). 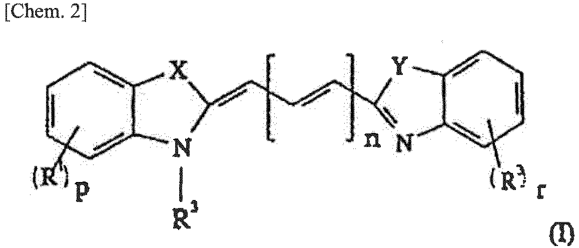 (wherein X and Y are selected individually out of >C(C₁-C₄alkyl)₂, sulfur, and oxygen; R¹ and R² are selected individually out of H, CH₂NH₂, SO₃⁻, phosphate, phosphonate, quaternary ammonium, NO₂, (CH₂)_{q}COOH, NCS, and CH₂NH-COR⁷, wherein R⁷ is a C₁-C₂₀ straight-chain or branched alkyl, or -(CH₂)_{q}-COOH, wherein q is an integer of 0 to 10; R³ is H or -L-P, wherein L is selected out of C₁-C₂₀ straight-chain or branched alkyls containing optionally 0, 1, or 2 unsaturated groups selected out of alkenyl, alkynyl, and aryl, and P is selected out of a reactive group, H, a C₁-C₂₀ straight-chain or branched alkyl, SO₃⁻, NH₂, quaternary ammonium, and CH₂NH-COR⁸, wherein R⁸ is a C₁-C₂₀ straight-chain or branched alkyl, -(CH₂)ₘ-COOH, or NHR⁹, wherein R⁹ is a C₁-C₂₀ straight-chain or branched alkyl, or COOH; n s an integer of 0 to 3; p and q are individually 0, 1, 2, 3, or 4, wherein insofar as p and /or r is larger than 1, each of R¹ and R² may be different, and m is an integer of 1 to 10.)

Specific examples of a dye compound expressed by Formula (I) are as disclosed in JP 2003-501540 W. The dyes have responsiveness to pH.

More specific examples of the environmentally-responsive dyes may include Fluo-3 produced by Life Technologies Corporation, and CypHer5E NHS ester produced by GE Healthcare Japan Corporation.

A combination of 2 or more environmentally-responsive dyes may be used, and a combination with another publicly known fluorescent substance dye (for example, a dye disclosed in JP 2015-59806A, JP 2015-93878 A, or JP 2015-108572 A) may be also used.

The accumulated amount of the environmentally-responsive dye is in a range from 200 to 10,000,000 molecules/particle, and preferably from 1500 to 2,000,000 molecules/particle. When the accumulated amount of a dye is in the range, the intensity of light emitted from a dye-accumulated nanoparticle is high, and fluorescence emitted corresponding to an ambient environment may be detected adequately by a detector.

Although it is possible to inject an environmentally-responsive dye directly into a cell, however by doing so, the dye is distributed uniformly in a cell, and therefore it is unable to evaluate a minute environment inside a cell. Further, in the case of a thermoplastic resin, probably due to difficulty in accumulation of a dye inside a particle, a dye inside a cell bleeds out so that a local environment may not be evaluated. Further, in the case of an inorganic particle such as silica, it is difficult to obtain a nanoparticle having accumulated a predetermined amount of dye. With respect to such particles, since accumulation of a dye may not necessarily be sufficient, an environment of a target limited part inside a cell may not be evaluated in detail.

### Method for producing environmentally-responsive dye-accumulated nanoparticle

There is no particular restriction on a method for producing the above environmentally-responsive dye-accumulated nanoparticle according to the present invention, insofar as the environmentally-responsive function as a fluorescent label is not impaired.

For example, it is possible to produce a dye-accumulated resin-made nanoparticle by polymerizing a source material for a resin as a source material for the thermosetting resin in the presence of an environmentally-responsive dye.

A usable source material for a resin may be a monomer corresponding to the thermosetting resin, or a prepolymer obtained from such a monomer.

When, for example, a melamine resin is used as a thermosetting resin, a melamine resin particle having accumulated an environmentally-responsive dye may be obtained by performing a polycondensation reaction by adding formic acid to a mixture liquid of the environmentally-responsive dye and a melamine resin. The reaction may be carried out, for example, in water. Further, if necessary, the polymerization reaction may be conducted in the presence of an appropriate surfactant. Further, an appropriate polymerization reaction promoter such as an acid may be added to a mixture liquid of the environmentally-responsive dye and a melamine resin for the purpose of promoting a polycondensation reaction for a thermosetting resin such as a melamine resin, and promoting an electrostatic interaction by adding a proton (H⁺) to a functional group such as an amino group contained in the resin or environmentally-responsive dye.

As a surfactant, heretofore well known various surfactants may be used, and an anion, a nonionic, a cationic, and an amphoteric surfactant may be included. Among them, a quaternary ammonium salt, such as tetrabutylammonium chloride or bromide, hexafluorophosphate, tetraoctylammonium bromide (TOAB), and tributylhexadecylphosphonium bromide, are preferable. Especially tetraoctylammonium bromide is preferable.

A reaction by a liquid phase method changes greatly depending on the condition of compounds including a solvent in the liquid. Production of a nano sized particle superior in monodispersity especially requires close attention. For example, in the case of a reversed micelle reaction method, the size or condition of a reversed micelle to act as a reaction field changes depending on the concentration or type of a surfactant, and therefore a window of conditions allowing formation of a nanoparticle is narrow. Consequently, an appropriate combination of a surfactant and a solvent becomes necessary.

The conditions of a polymerization reaction (temperature, time, etc.) may be set considering the type of a resin, the composition of a source material mixture, etc. With respect to synthesis of a thermosetting resin such as a melamine resin, the reaction temperature is ordinarily 60 to 200°C, and the reaction time is ordinarily 20 to 120 min. It is appropriate to set the reaction temperature at a temperature, at which the performance of a fluorescent dye is not deteriorated (within the heat-resistant temperature range). Heating may be divided into a plurality of steps. For example, a reaction may be conducted at a relatively low temperature for a certain time period, and then the temperature is raised to conduct a reaction at a relatively high temperature for a certain time period.

After the end of a polymerization reaction, impurities, such as a surplus source material for a resin, an environmentally-responsive dye, and a surfactant, should be removed from the reaction solution, and a formed particle should be recovered and purified. For example, the reaction solution is centrifuged, the supernatant containing impurities is removed, and then the residue is reslurried and washed by addition of ultrapure water with ultrasonic irradiation. The procedure is preferably repeated plural times until light absorption, or fluorescence attributed to a resin or a dye becomes undetectable in the supernatant.

### (Regulation of average particle diameter)

In a case in which a dye particle is synthesized by an emulsion polymerization method, a micelle with an aqueous phase on the outer side and an oil phase in the inner side is formed by a surfactant in a reaction system, so that a monomer forming the resin is contained in the oil phase in the inner side of the micelle, and a polymerization reaction occurs inside the micelle.

For example, when the dye particle is synthesized, by adding a surfactant having an emulsification action in a range of 10 to 60 weight-% with respect to a source material for a resin, any optional particle diameter may be obtained such that a particle between, for example, 30 nm and 300 nm may be produced. If the percentage of a surfactant is increased, a smaller particle may be produced, and even a particle of 30 nm or smaller is producible. Conversely, if the percentage of a surfactant is decreased, a larger particle may be produced, and even a particle of 300 nm or larger is producible. With a constant amount of a used surfactant, the average particle diameter of a dye particle may be also regulated by changing the respective percentages of a source material for a resin and a fluorescent substance to be used for producing a dye particle with respect to the entire reaction system.

### (Surface modification)

According to the present invention an obtained environmentally responsive dye accumulated nanoparticle may be used as it is, however, if necessary, an environmentally-responsive dye-accumulated nanoparticle of the present invention may be subjected to surface modification.

Although there is no particular restriction on surface modification to be performed according to the present invention, it should be performed preferably by introduction of a functional group able to form a bond with another molecule.

Examples of a "functional group able to form a bond with another molecule" may include functional groups used generally in the biochemical field, and specific examples of such functional groups include a hydroxy group, an amino group, a carboxyl group, a thiol group, a maleimide group, and an aldehyde group. A "functional group able to form a bond with another molecule" may be hereinafter occasionally referred to as "reactive functional group").

Specifically, an environmentally-responsive dye-accumulated nanoparticle superior in a property of cellular uptake may be provided by coating the surface of a nanoparticle with poly(ethylene glycol) chains.

Although there is no particular restriction on a poly(ethylene glycol) capable of coverage with poly(ethylene glycol) chains insofar as it is a compound having poly(ethylene glycol) chains, specific examples thereof may include a poly(ethylene glycol), such as HS-C₂H₄(OCH₂CH₂)ₙ-OCH₃, NH₂-C₂H₄-(OCH₂CH₂)ₙ-OCH₃, C(=O)H-C₂H₄-(OCH₂CH₂)ₙ-OCH₃, NH₂-C₂H₄-(CH₂CH₂O)ₙ-OC(=O)O-succinimide, maleimide-(CH₂)₂C(=O)NHC₃H₆-(CH₂CH₂O)ₙ-OC(=O)O-succinimide, HO-(CH₂CH₂O)ₙ-CH₂CH₂C(=O)H, HO-(CH₂CH₂O)ₙ-C₃H₆NH₂, H₂N(CH₂)₃₀(CH₂CH₂O)ₙ(CH₂)₅C(=O)OH, and biotin-(CH₂)₄C(=O)NHC₃H₆(CH₂CH₂O)ₙ-O-C(=O)O-succinimide.

With respect to bonding between a poly(ethylene glycol) chain and an environmentally-responsive dye-accumulated nanoparticle, a poly(ethylene glycol) may be bonded directly to a semiconductor nanoparticle, or it may be bonded by the intermediary of an organic substance such as a silane coupling agent. In a case in which a poly(ethylene glycol) chain has a carboxy group at the end, the organic substance is a compound having a functional group able to bond with a carboxy group, and examples of the functional group may include an amino group, a mercapto group, and a hydroxy group. In a case in which a poly(ethylene glycol) chain has an amino group at the end, a functional group of the organic substance may be a carboxyl group, etc. A preferable coupling agent is an aminotrialkoxysilane such as 3-aminopropyltriethoxysilane (APS).

### [Use of environmentally-responsive dye-accumulated nanoparticle]

An environmentally-responsive dye-accumulated nanoparticle according to the present invention may be used as a reagent for an intracellular environmental analysis, and is applicable to a method for analyzing intracellular environment, by which such an environmentally-responsive dye-accumulated nanoparticle is introduced into a cell so as to evaluate the inside of the cell.

An intracellular environment includes as described above pH, ion concentration, gas concentration, pressure, etc. and a dye is selected corresponding to a target environment to be investigated.

In the analysis method an environmentally-responsive dye-accumulated nanoparticle is added a culture solution of a cultured cell, and the environmentally-responsive dye-accumulated nanoparticle is introduced into a cell by performing incubation. For facilitating introduction of a nanoparticle into a cell, a surfactant may be added. After the incubation for a predetermined time period, a cultured cell is observed under a fluorescence microscope to observe fluorescence intensity attributable to each particle. The difference in an environment may be evaluated based on the fluorescence intensity of each particle and a calibration curve prepared in advance.

### Examples

The present invention will be described below by way of Examples, provided that the present invention be not restricted in any way by the Examples.

### [Example 1]

### Melamine resin nanoparticle having accumulated dye responsive to Ca ion

As a dye responsive to a Ca ion, 1 mg of Fluo-3 produced by Life Technologies Corporation was added into 1 mL of water and dissolved. Then 2 mL of a 5% aqueous solution of Emulsion 430 (registered trademark), an emulsifier for emulsification polymerization (polyoxyethylene oleyl ether, produced by Kao Corporation), was added to the solution. The solution was stirred on a hot plate stirrer and the temperature was elevated to 70°C, and then 0.8 g of a source material for a melamine resin NIKALAC MX-035 (produced by Nippon Carbide Industries Co., Inc.) was added to the solution.

To the solution 200 µL of a 10% aqueous solution of dodecylbenzenesulfonic acid (produced by Kanto Chemical Co., Ltd.) was added as a surfactant, and the solution was heated with stirring at 70°C for 50 min. Then the temperature was elevated to 90°C and maintained for 20 min with heating and stirring. The obtained particle-dispersion liquid was washed with pure water to remove impurities, such as a surplus source material for a resin, a dye, etc. For washing, the liquid was centrifuged with a centrifuge (Micro-refrigerated centrifuge 3740, produced by Kubota Corporation) at 20000 G for 15 min, the supernatant was removed, and then the residue was reslurried by addition of ultrapure water with ultrasonic irradiation. Washing by centrifugation, removal of a supernatant, and redispersion in ultrapure water was repeated five times. The average particle diameter of the obtained dye-accumulated nanoparticle was 200 nm.

Ethylene glycol (Methyl-PEG₁₂-NHS Ester, Tokyo Chemical Industry Co., Ltd.) was added to the obtained dye-accumulated nanoparticle up to 0.1 mol/L allowing a reaction for 12 hours.

### [Comparative Example 1]

A 14 mass-% ammonia water was further diluted 5-fold with ethanol to prepare 100 mL of a solvent containing ammonia water. Into the solvent 500 µL-volume of TEOS (tetraethylorthosilicate) to occupy 0.5 volume-%, and a DMF solution, prepared by adding a solution, in which 1 mg of the environmentally-responsive Fluo-3 produced by Life Technologies Corporation and used in Example 1 having the same volume as the TEOS was dissolved in 1 mL of water, into DMF, were added respectively, and the liquid was stirred at room temperature (25°C).

Formation of silica colloid was almost complete after stirring for 1 hour, and Fluo-3 was introduced in a silica particle after 5 hours from the initiation of a reaction.

Into the obtained silica particle dispersion liquid, TEOS was additionally charged, and the mixture was made to react at room temperature for 5 hours to yield a silica nanoparticle with a silica layer. The amount of additionally charged TEOS was 50% of the amount of initially charged TEOS.

The silica nanoparticle dispersion liquid obtained as above was centrifuged for 30 min (5000×g) to precipitate a particle, and the supernatant was removed immediately. The obtained precipitate was reslurried in ethanol, and centrifuged again (5000×g) for 30 min to precipitate a particle. The same ethanol washing operation was repeated once more to remove unreacted TEOS, etc. Further, the same washing operation except that distilled water was used instead of ethanol was conducted four times to remove a free dye, etc. The average particle diameter of the obtained dye-accumulated silica nanoparticle was 200 nm. The surface of the prepared dye-accumulated silica nanoparticle was also modified with poly(ethylene glycol) identically with Example 1.

### [Comparative Example 2]

With respect to a polystyrene particle a commercial product (produced by Merck & Co., Inc.) was used. The average particle diameter of the polystyrene particle is 200 nm, and the surface functional group is an amino group.

The polystyrene particle was dispersed in 10 mL of methanol, and to the dispersion a solution, for which 1 mg of Fluo-3 produced by Life Technologies Corporation was added to 1 mL of dichloromethane and dissolved, was added and the dispersion was stirred for 3 hours. Dichloromethane was distilled away under reduced pressure. Centrifugation was conducted for 30 min to precipitate a particle, and the supernatant was removed immediately. The obtained precipitate was reslurried in water, and centrifuged again to precipitate a particle. The average particle diameter of the obtained dye-accumulated silica nanoparticle was 200 nm.

The surface of the prepared dye-accumulated polystyrene nanoparticle was also modified with poly(ethylene glycol) identically with Example 1.

### Evaluation 1

The dependence of fluorescence intensity on Ca ion was evaluated with respect to dye-accumulated nanoparticles prepared in Example 1, and Comparative Examples 1 and 2.

A fluorescence intensity measurement in a state where a Ca ion was absent was conducted with a 0.1 nM dispersion of particles in a phosphate buffer solution (pH 6.9). A fluorescence intensity measurement in the presence of a Ca ion was conducted with a 0.1 nM dispersion of particles in a citrate buffer solution (pH 6.9) containing 10 mM of Ca ion.

With respect to a measurement of fluorescence intensity, a fluorescence intensity at wavelength 580 nm was measured using an excitation light wavelength of 488 nm with a fluorescence spectrophotometer F-7000 (trade name, produced by Hitachi High-Technologies Corporation).

**[Table 1]**

| | Example 1 | Comparative Example 1 | Comparative Example 2 |
|---|---|---|---|
| Nanoparticle | Particle 1 | Particle 2 | Particle 3 |
| Dye responsive to Ca ion | Fluo-3 | Fluo-3 | Fluo-3 |
| Nanoparticle material | Melamine resin (thermosetting resin) | Silica (inorganic material) | Polystyrene (thermoplastic resin) |
| Average particle diameter (nm) | 200 | 200 | 200 |
| (molecules/particle) | 50,000 | 6,000 | 3,000 |
| Fluorescence intensity without Ca ion | 30 | 5 | 2 |
| Fluorescence intensity with Ca ion | 300 | 45 | 25 |
| Ratio of Fluorescence intensity with Ca ion / Fluorescence intensity without Ca ion | 10 | 9 | 13 |

It is obvious that a particle according to the present invention changes its fluorescence intensity corresponding to existence or nonexistence of a Ca ion, and that its fluorescence intensity is higher than a conventional particle.

### [Example 2]

### Melamine resin nanoparticle having accumulated dye responsive to pH

A polymerization reaction for a melamine resin was performed in the presence of a dye identically with Example 1 using CypHer5E NHS ester produced by GE Healthcare Japan Corporation as a dye responsive to pH to synthesize a melamine resin nanoparticle with an average particle diameter of 100 nm.

The surface was modified with poly(ethylene glycol) identically with Example 1 to synthesize particle 4.

### Evaluation 2

Respective dispersions of the particle 4 in buffer solutions of pH 3, 4, 5, 6, and 7 were placed on a slide and observed under a fluorescence microscope. A calibration curve showing correlation between pH and fluorescence intensity was prepared.

Next, to a DMEM (Dulbecco's modified Eagle medium, produced by Sigma-Aldrich) culture solution of a cultured cell, 100 µL of a 50 pM particle 4 dispersion, and 50 µL of a surfactant Tween 20 (produced by Tokyo Chemical Industry Co., Ltd.) were added and the liquid was incubated at 37°C for 5 hours. An incubated cell was observed using a fluorescence microscope. Fluorescence attributed to a particle was observed as a dot in a cell. From the fluorescence intensity of each particle, the pH around a particle could be determined based on the calibration curve prepared separately.

**[Table 2]**

| | Example 2 |
|---|---|
| Stain | Particle 4 |
| Dye responsive to pH | CypHer5E NHS ester |
| Accumulating material | Melamine resin |
| | (thermosetting resin) |
| Average particle diameter (nm) | 100 |
| Accumulated amount of dye | 15000 molecules/particle |
| Fluorescence intensity under neutral condition | 50 |
| Fluorescence intensity under acidic condition | 300 |
| Intracellular observation | Fluorescence observed as dot |

### [Example 3]

### Melamine resin nanoparticle having accumulated dye responsive to Ca ion

As a dye responsive to a Ca ion, 1 mg of Fluo-3 produced by Life Technologies Corporation was added into 1 mL of water and dissolved. Then 2 mL of a 5% aqueous solution of 430 (registered trademark), an emulsion of an emulsifier for emulsification polymerization (polyoxyethylene oleyl ether, produced by Kao Corporation) was added to the solution. The solution was stirred on a hot plate stirrer and the temperature was elevated to 70°C, and then 0.2 g of a source material for a melamine resin NIKALAC MX-035 (produced by Nippon Carbide Industries Co., Inc.) was added to the solution.

To the solution 200 µL of a 10% aqueous solution of dodecylbenzenesulfonic acid (produced by Kanto Chemical Co., Ltd.) was added as a surfactant, and the solution was heated with stirring at 50°C for 50 min. Then the temperature was elevated to 90°C and maintained for 20 min with heating and stirring. The obtained particie-dispersion liquid was washed with pure water to remove impurities, such as a surplus source material for a resin, a dye, etc. For washing, the liquid was centrifuged with a centrifuge (Micro-refrigerated centrifuge 3740, produced by Kubota Corporation) at 100000 G for 15 min, the supernatant was removed, and then the residue was reslurried by addition of ultrapure water with ultrasonic irradiation. Washing by centrifugation, removal of a supernatant, and redispersion in ultrapure water was repeated five times. The average particle diameter of the obtained dye-accumulated nanoparticle was 30 nm.

Ethylene glycol (Methyl-PEG₁₂-NHS Ester, Tokyo Chemical Industry Co., Ltd.) was added to the obtained dye-accumulated nanoparticle up to 0.1 mol/L allowing a reaction for 12 hours.

### [Example 4]

### Melamine resin nanoparticle having accumulated dye responsive to Ca ion

As a dye responsive to a Ca ion, 1 mg of Fluo-3 produced by Life Technologies Corporation was added into 1 mL of water and dissolved. Then 2 mL of a 5% aqueous solution of 430 (registered trademark), an emulsion of an emulsifier for emulsification polymerization (polyoxyethylene oleyl ether, produced by Kao Corporation) was added to the solution. The solution was stirred on a hot plate stirrer and the temperature was elevated to 70°C, and then 1.2 g of a source material for a melamine resin NIKALAC MX-035 (produced by Nippon Carbide Industries Co., Inc.) was added to the solution.

To the solution 200 µL of a 10% aqueous solution of dodecylbenzenesulfonic acid (produced by Kanto Chemical Co., Ltd.) was added as a surfactant, and the solution was heated with stirring at 70°C for 50 min. Then the temperature was elevated to 90°C and maintained for 20 min with heating and stirring. The obtained particle-dispersion liquid was washed with pure water to remove impurities, such as a surplus source material for a resin, a dye, etc. For washing, the liquid was centrifuged with a centrifuge (Micro-refrigerated centrifuge 3740, produced by Kubota Corporation) at 20000 G for 15 min, the supernatant was removed, and then the residue was reslurried by addition of ultrapure water with ultrasonic irradiation. Washing by centrifugation, removal of a supernatant, and redispersion in ultrapure water was repeated five times. The average particle diameter of the obtained dye-accumulated nanoparticle was 800 nm.

Ethylene glycol (Methyl-PEG₁₂-NHS Ester, Tokyo Chemical Industry Co., Ltd.) was added to the obtained dye-accumulated nanoparticle up to 0.1 mol/L allowing a reaction for 12 hours.

### [Comparative Example 3]

### Melamine resin nanoparticle having accumulated dye responsive to Ca ion

As a dye responsive to a Ca ion, 1 mg of Fluo-3 produced by Life Technologies Corporation was added into 1 mL of water and dissolved. Then 2 mL of a 5% aqueous solution of 430 (registered trademark), an emulsion of an emulsifier for emulsification polymerization (polyoxyethylene oleyl ether, produced by Kao Corporation) was added to the solution. The solution was stirred on a hot plate stirrer and the temperature was elevated to 70°C, and then 0.1 g of a source material for a melamine resin NIKALAC MX-035 (produced by Nippon Carbide Industries Co., Inc.) was added to the solution.

To the solution 200 µL of a 10% aqueous solution of dodecylbenzenesulfonic acid (produced by Kanto Chemical Co., Ltd.) was added as a surfactant, and the solution was heated with stirring at 50°C for 50 min. Then the temperature was elevated to 70°C and maintained for 20 min with heating and stirring. The obtained particle-dispersion liquid was washed with pure water to remove impurities, such as a surplus source material for a resin, a dye, etc. For washing, the liquid was centrifuged with a centrifuge (Micro-refrigerated centrifuge 3740, produced by Kubota Corporation) at 100000 G for 60 min, the supernatant was removed, and then the residue was reslurried by addition of ultrapure water with ultrasonic irradiation. Washing by centrifugation, removal of a supernatant, and redispersion in ultrapure water was repeated five times. The average particle diameter of the obtained dye-accumulated nanoparticle was 20 nm.

Ethylene glycol (Methyl-PEG₁₂-NHS Ester, Tokyo Chemical Industry Co., Ltd.) was added to the obtained dye-accumulated nanoparticle up to 0.1 mol/L allowing a reaction for 12 hours.

The dependence of fluorescence intensity on Ca ion was evaluated with respect to dye-accumulated nanoparticles prepared in Examples 3 and 4, and Comparative Example 3 identically with Evaluation 1.

**[Table 3]**

| | Example 3 | Example 4 | Comparative Example 3 |
|---|---|---|---|
| Nanoparticle | Particle 5 | Particle 6 | Particle 7 |
| Dye responsive to Ca ion | Fluo-3 | Fluo-3 | Fluo- 3 |
| Nanoparticle material | Melamine resin (thermosetting resin) | Melamine resin (thermosetting resin) | Melamine resin (thermosetting resin) |
| Average particle diameter (nm) | 30 | 800 | 20 |
| Accumulated amount of dye (molecules/particle) | 200 | 10,000,000 | 100 |
| Fluorescence intensity without Ca ion | 0.12 | 5400 | 0.04 |
| Fluorescence intensity with Ca ion | 1.2 | 60,000 | 0.5 |
| Ratio of Fluorescence intensity with Ca ion / Fluorescence intensity without Ca ion | 10 | 11 | 12 |

## Claims

1. An environmentally-responsive dye-accumulated nanoparticle, in which an environmentally-responsive dye is accumulated on the surface of, or inside a thermosetting resin particle, and which has an average particle diameter of 30 to 800 nm, wherein the accumulated amount of the environmentally-responsive dye is 200 to 10,000,000 molecules/particle.

2. The environmentally-responsive dye-accumulated nanoparticle according to claim 1, wherein the environmentally-responsive dye has dependence on pH, or a Ca ion in terms of fluorescence intensity.

3. The environmentally-responsive dye-accumulated nanoparticle according to claim 1 or 2, wherein the thermosetting resin is a melamine resin.

4. The environmentally-responsive dye-accumulated nanoparticle according to any one of claims 1 to 3, wherein the surface is further modified with poly(ethylene glycol).

5. A reagent for an intracellular environment analysis comprising the environmentally-responsive dye-accumulated nanoparticle according to any one of claims 1 to 4.

6. A method for analyzing intracellular environment, wherein the environmentally-responsive dye-accumulated nanoparticle according to any one of claims 1 to 4 is introduced into a cell, and the inside of the cell is evaluated.
